Europäisches Patentamt

European Patent Office

Office européen des brevets

(11) Publication number: 0 265 177
A2

# EUROPEAN PATENT APPLICATION

(21) Application number: 87309120.1

(22) Date of filing: 15.10.87

(51) Int. Cl.4: G01N 33/68 , C07K 3/18 , B01D 15/08 , G01N 33/577 , G01N 33/50

(30) Priority: 17.10.86 US 920588

(43) Date of publication of application:
27.04.88 Bulletin 88/17

(84) Designated Contracting States:
AT BE CH DE ES FR GB GR IT LI LU NL SE

(71) Applicant: INTERFERON SCIENCES, INC.
783 Jersey Avenue
New Brunswick New Jersey 08901(US)

(72) Inventor: Rybacek, Ludvik
22 Rolling Road
Somerset N.J. 08873(US)
Inventor: Tarnowski, Stanley J. Jr.
252 Lanitos Avenue
Sunnyvale California(US)
Inventor: D'Andrea, Mark J.
70C Montgomery Road
Neshanic Station N.J. 08853(US)

(74) Representative: Bannerman, David Gardner et al
Withers & Rogers 4 Dyer's Buildings Holborn
London, EC1N 2JT(GB)

(54) Interferon assay.

(57) A rapid assay for quantifying the amounts of interferon in liquid samples is provided. The assay employs an affinity column, containing immobilized anti-interferon antibodies, in series with a reversed-phase column. The assay is able to measure interferon concentrations as low as 1-2 micrograms/milliliter in total assay times of less than one hour. By suitably adjusting the elution gradient used with the reversed-phase column, different interferon subtypes, e.g., monomeric and oligomeric forms of $\alpha 2$ interferon, can be separately detected and quantified. In certain preferred embodiments, errors due to non-specific binding are minimized by treating the affinity column with an albumin-containing buffer prior to each assay.

## INTERFERON ASSAY

### BACKGROUND OF THE INVENTION

#### 1. Field of the Invention

This invention relates to interferon and in particular to an improved assay for quantifying the amounts of interferon in liquid samples.

#### 2. Other Interferon Assays

Since its discovery in 1957 by Isaacs and Lindenmann, interferon has been the subject of extensive research investigations. As a result of these efforts, both natural and recombinant interferons have now become commercial products. In parallel with this ever increasing interest in interferon, there has been a continuing demand for new and improved assays for interferon. Such assays are needed both to understand and control the manufacturing processes used to prepare interferon and to quantify and characterize the finished product.

The standard assay currently in use to measure amounts of interferon in liquid samples is the cytopathic effect (CPE) inhibition assay. A general description of the format employed in this assay appears in U.S. Pat. No. 4,241,174 to Familletti et al., entitled "Interferon Assay".

Briefly, the assay involves incubating cells known to be sensitive to interferon with a serial dilution of the sample whose titer is to be determined, adding a challenge virus to each of the dilutions, incubating again, and then observing the level of dilution which achieves a 50% reduction in the virus' cytopathic effect on the cells. Concentrations in terms of standard units are determined by simultaneously performing the assay on international standards, such as those established by the National Institutes of Health, Bethesda, Maryland.

As has been recognized by persons skilled in the art, the CPE inhibition assay suffers from a variety of problems. First, since it uses living cells, it is susceptible to errors due to biological variability even in the hands of a single investigator. Indeed, the precision of the assay is typically on the order of ±30%, and can be as low as ±50% relative to the value measured. Second, performance of the assay at a minimum takes about 24 hours, and typically takes between about 96 and 120 hours. Plainly, assay times of this length make this assay unsuitable for real time monitoring of interferon production processes. Further, the assay only provides information regarding total interferon activity and gives no information about different forms and types of interferon which may be present in the sample being assayed.

In an effort to overcome some of the problems with the CPE inhibition assay, other assay techniques have been proposed. For example, solid phase, sandwich radioimmunoassays have been employed to measure interferon quantities. See Staehelin et al., "A Rapid Quantitative Assay of High Sensitivity for Human Leukocyte Interferon with Monoclonal Antibodies", Methods Enzymol., Vol. 79, pages 589-595, 1981. Although this technique does reduce the amount of time needed to perform an assay and does reduce the effects of biological variability, sandwich radioimmunoassays are complex assays to perform and, as practiced by Staehelin et al., a single assay will not provide information about different forms and types of interferon present in a sample.

Similarly, immobilized antibody columns, originally used to purify interferon, have been adapted to perform assays for interferon. See Roy et al., "Automated High-Performance Immunosorbent Assay for Recombinant Leukocyte A Interferon," Journal of Chromatography, Vol. 327, pages 189-192, 1985. As with radioimmunoassays, this assay does improve on the CPE inhibition assay with regard to biological variability and assay time. However, the antibody column assay still cannot distinguish different interferon components in a mixture. Also, this assay has relatively low sensitivity, e.g., the lowest concentrations measurable are on the order of 30 micrograms of interferon per milliliter of sample.

Recently, Pestka et al, have described a sandwich radioimmunoassay which is said to detect oligomers of alpha interferon in a mixture of monomers and oligomers. See Pestka et al., "Procedures for Measurement of Interferon Dimers and Higher Oligomers by Radioimmunoassay", Methods in Enzymology, vol. 119, pages 588-593, 1986. The assay uses a bound and radiolabelled version of an antibody which recognizes a single epitope, and requires that the oligomer which is to be detected have at least two epitope sites available for recognition by the antibody. Significantly, this assay does not detect inteferon monomers, for which a separate assay must be performed.

## SUMMARY OF THE INVENTION

In view of the foregoing state of the art, it is an object of the present invention to provide improved assays for measuring the amounts of interferon in liquid samples. More particularly, it is an object of the invention to provide assays for measuring interferon concentrations which are simple to perform, insensitive to biological variability, rapid, and which have sensitivities below 30 ug/ml. Even more particularly, it is an object of the invention to provide interferon assays which are able to separate and quantify the amounts of different types or forms of interferon in a liquid sample.

It is a further object of the invention to provide interferon assays which are suitable for use in monitoring and/or controlling industrial processes for producing commercial quantities of interferon both from natural sources and through the use of recombinant DNA techniques.

To achieve the foregoing and other objects, the invention provides a method for detecting interferon in a solution comprising the steps of:

(a) passing the solution through an affinity column containing an anti-interferon antibody coupled to a solid support whereby a portion of the interferon in the solution becomes bound to the antibody;

(b) passing a wash buffer through the column to remove non-interferon substances in the solution from the column without removing a substantial amount of the interferon which has become bound to the antibody;

(c) passing an eluting buffer through the column to disrupt the interferon-antibody complex and thus form an intermediate solution which contains a portion of the previously bound interferon dissolved in the eluting buffer;

(d) passing the intermediate solution through a reversed-phase column containing a solid support to which is bonded a hydrophobic ligand whereby a portion of the interferon in the intermediate solution is separated from the solution and becomes bound to the column;

(e) eluting the bound interferon from the reversed-phase column by passing an increasing gradient of one or more organic solvents through the column; and

(f) sensing the amount of protein in the eluate from the reversed-phase column as a function of time.

As discussed in detail below in connection with the description of the preferred embodiments, if desired, additional washings and equilibrations of the affinity and reversed-phase columns can be performed at appropriate stages of the process.

In connection with certain preferred embodiments, the amount of interferon in the solution is quantified by comparing the amount of protein sensed in step (f) with a standard curve obtained by performing steps (a) through (f) on solutions containing known amounts of interferon. In contrast to the prior art, by using an affinity column in series with a reversed-phase column, it has been found that quantities of interferon well below 30 micrograms/milliliter, e.g., on the order of 1-2 micrograms/milliliter, can be accurately measured in this way, with the total assay taking substantially less than an hour to perform.

In connection with other preferred embodiments, interferon subcomponents within the solution which have bound to the affinity column can be identified and separately quantified by adjusting the gradient of the organic solvent used to elute the interferon from the reversed-phase column. For example, as demonstrated in Example 2 below, three different forms of recombinant α2 interferon, i.e., a monomeric form having intact disulfide bonds, a monomeric form having some disulfide bonds broken, and an oligomeric form, can be readily made to elute from the reversed-phase column at different times by suitably adjusting the gradient of the organic solvent.

In terms of commercial production of recombinant α2 interferon, this ability to distinguish between different interferon forms is of particular importance since certain interferon forms, e.g., the monomeric form having some disulfide bonds broken, may be less pharmaceutically acceptable than other forms, e.g., may result in more adverse reactions in human subjects than other forms. By means of the present invention, the relative amounts of the various forms existing at different stages in the production process can be readily determined or monitored. Moreover, in view of the short times required to perform the assay, these determinations can be made in real time as the process is being performed. Based on this knowledge, which was not previously available to the art, the overall process can be optimized to produce the maximum amounts of the most effective forms of interferon.

In connection with the foregoing embodiments, it is further preferred to use silica particles as the solid support for the anti-interferon antibody used in the affinity column. In connection with the use of this support, it has been found preferably to wash the column with an albumin-containing buffer, e.g., a buffer containing bovine serum albumin (BSA). As discussed in more detail below, it is believed that the BSA binds to silanol groups which result from mechanical or chemical perturbation of the column matrix during its manufacture or use, and thus reduces the level of non-specific binding of interferon and other proteins to the affinity column. More generally, proteins other than albumin can be used to perform the blocking function. Such proteins must have the characteristic that they elute from the reversed-phase column at a different time than the interferon being assayed elutes from that column so as not to interfere with the detection of the interferon. Also, such proteins must be able to react with silanol groups. In general, the proteins will have isoelectric points, molecular weights and hydrophobicities similar to that of albumin.

The principles of the invention, as well as its preferred embodiments, are explained and illustrated by the accompanying figures and the examples presented below. These figures and examples, of course, are for purposes of illustration only and are not intended to limit the scope of the invention.

BRIEF DESCRIPTION OF THE DRAWINGS

Figure 1 is a schematic diagram of apparatus suitable for performing the assay of the present invention.

Figure 2 illustrates the effects of different reversed-phase elution profiles on the ability of the assay to separately detect and quantify different interferon subtypes.

Figure 3 is a collection of UV absorbance versus time tracings generated by the assay of the present invention.

Figure 4 is a standard curve for quantifying $\alpha 2$ interferon concentrations in accordance with the present invention.

DESCRIPTION OF THE PREFERRED EMBODIMENTS

As discussed above, the present invention is directed to a high sensitivity, rapid assay for interferon which employs an affinity column in series with a reversed-phase (RP) column.

The assay is applicable to all types of interferons including natural interferons, interferons produced by recombinant DNA technology, interferons produced by chemical synthesis, and interferons derived from naturally occurring interferons by, for example, site specific mutagenesis. Examples of the more common types of interferons which can be assayed with the invention include alpha, beta and gamma interferons of human and animal origin.

Samples to be assayed can contain a single type of interferon or mixtures of various types of interferons. Further, as discussed above, the sample can contain a mixture of different interferon subtypes, such as, for example, monomer and polymer forms of alpha interferon, monomer and polymer forms of alpha interferon, monomer and dimer forms of beta interferon, and monomer and polymer forms of gamma interferon. The samples to be assayed can come directly from the production process used to produce the interferon without any prior purification, or can come from the final product after substantial purification has been performed.

In accordance with the invention, only minimal preparation of samples to be assayed is required. Thus, samples should be clarified to remove solid materials, such as cell debris, which could clog the components of the system or the columns. The clarification can conveniently be performed by, for example, centrifuging and/or filtering the samples. Besides clarification, the only other requirement is that the pH of the sample be in a range in which the anti-interferon antibody will bind to the interferon in the sample. In general, pH's in the range of 5 to 9, and preferably in the range of 6 to 8, are suitable for the commonly available monoclonal and polyclonal anti-interferon antibodies.

The affinity column used in the assay consists of an anti-interferon antibody bound to a solid support. A variety of monoclonal and polyclonal anti-interferon antibodies known in the art can be used in the practice of the invention. For example, in the case of assays for human recombinant $\alpha 2$ interferon, the monoclonal antibody LIT-1, developed by Interferon Sciences, Inc., New Brunswick, New Jersey, and manufactured by Charles Rivers Biotechnical Services, Inc., Wilmington, Massachusetts, have been found to work successfully.

Typically, the assay will be directed to a single type of interferon, e.g., recombinant $\alpha 2$ interferon, and only antibodies capable of binding to that type will be used on the column. However, if a simultaneous assay for more than one type of interferon is desired, a mixture of antibodies against different interferon types can be used.

Various solid supports can be used in the affinity column. Examples include cross-linked agarose, polymer-silica, hydroxyalkylmethacrylate, titanium hydroxide-silica, oxirane acrylic beads (Eupergit), polyacrylamide agarose, cellulosic materials, and porous, polyhydroxylated silica particles. Porous, polyhydroxylated silica particles are, in general, preferred since they are not subject to compression at high operating pressures. High operating pressures, in turn, mean higher flow rates through the affinity column, and thus shorter assay times.

A variety of coupling techniques known in the art can be used to attach the antibody to the solid support. Examples include periodate activation of polyhydroxylated silica, carbonyl diimidozole, cyanogen bromide, oxirane and other activation methods. After coupling has been completed, a blocking agent, such as ethanolamine, can be used to block activated sites on the solid support which have not reacted with antibody. If not blocked, such unreacted sites can lead to high levels of non-specific binding to the column of non-interferon proteins present in the sample to be assayed. These non-interferon proteins can, in turn, lead to erroneous assay results if they and the interferon being assayed are released together from the affinity column.

In the case of solid supports composed of polyhydroxylated silica, it has been found that some residual silanol groups continue to exist on the surface of the particles even after the polyhydoxylation process has been completed. In addition, new silanol groups are formed by fracturing of the silica particles during the antibody coupling procedure and by slow dissolution of the particles over time as many assays are performed. It is believed that these silanol groups lead to non-specific binding of proteins to the affinity column.

In accordance with the invention, a method has been found for eliminating this residual non-specific binding. As discussed above, the method involves treating the solid support with an albumin-containing buffer, e.g., a buffer containing bovine serum albumin (BSA) or human serum albumin (HSA). The albumin concentration in the buffer should typically be on the order of between about 0.01 mg/ml and about 5 mg/ml, and preferably on the order of between about 0.1 mg/ml and about 1 mg/ml.

The albumin in the buffer reacts with the residual silanol groups, thus preventing interferon and non-interferon proteins in the sample from reacting with these groups. To the extent that some of the albumin elutes from the affinity column with the interferon, it has been found that such albumin elutes from the reversed-phase column before interferon elutes, and thus does not interfere with the interferon analysis. In general, it is preferred to wash the affinity column with the albumin-containing buffer in connection with each performance of the assay. In addition to the albumin treatment, it has also been found beneficial to remove fine particles of silica either prior to or after the antibody coupling process.

As with the affinity column, various reversed-phase columns, capable of binding interferon, can be used with the present invention. For example, reversed-phase columns containing $C_3$, $C_4$, $C_5$, $C_6$, $C_{18}$, and phenyl groups bound to various types of solid supports, e.g., agarose or silica supports, can be used. Columns of these types are commercially available from various manufacturers, including The Separation Group, Hesperia, CA, Du Pont Co., Willmington, DE, Beckman Instruments, San Ramon, CA, J. T. Baker, Philipsburg, NJ.

Similarly, various combinations of aqueous and organic solvents can be used to elute the interferon from the reversed-phase columns. Examples include a mixture of trifluoroacetic acid (TFA) with water (aqueous solvent) in combination with a mixture of TFA with acetonitrile (organic solvent), low alkyl alcohols, glycols and non-ionic detergents. The TFA/water plus TFA/acetonitrile combination is preferred since it has been found that interferon activity is generally not lost in the presence of these solvents. Also, acetonitrile/TFA/water solutions can be readily volitalized and thus separated from interferon, again without significant losses in interferon activity. In addition, the same type of buffers, e.g., a TFA-based, acidic buffer, can be used to elute interferon from the affinity column. Moreover, TFA is relatively non-corrosive to the various valves, pumps, and related equipment used to perform the assay (see discussion below).

The amount of interferon leaving the reversed-phase column as a function of time can be monitored in various ways, the preferred approach being optical monitoring using, for example, a light source having a wavelength which is absorbed by proteins, e.g., a 280 nm UV light source whose output is absorbed by the amino acids, tyrosine, phenylalanine, and tryptophan.

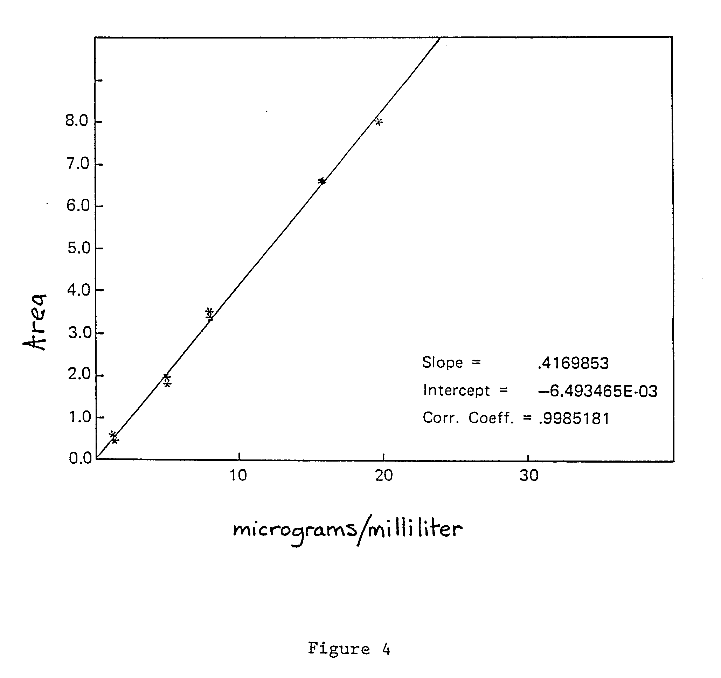

To obtain quantitative measures of the amount of interferon in a sample, the output of the monitor is fed to an integrator which sums the area under, for example, the absorbance versus time graph generated by the monitor. This integrated value is then transformed to a mass per volume value through the use of a standard curve generated from integrated values obtained by performing the assay on known quantities of interferon of the type being assayed (see, for example, the standard curve of Figure 4).

Total interferon concentrations are determined by integrating the full absorbance versus time graph and comparing that value with the standard curve. Concentrations of individual interferon subtypes are determined by limiting the integration to the portion of the absorbance graph corresponding to the subtype and comparing that value with the standard curve. If interferon types having different absorption characteristics are to be analyzed simultaneously, a standard curve for each interferon type is prepared, the area under the absorbance curve for each interferon type is computed separately, and each area is compared with the appropriate standard curve for that type of interferon. To perform this analysis, the different types of interferon must elute from the reversed-phase column at different times.

Although the assay of the present invention can be performed manually, in its preferred embodiments, the assay is automated through the use of electronically controlled switching valves, pumps, and gradient generators. Suitable equipment of this type is available from various manufacturers, including, the Waters Chromatography Division of Millipore Corporation, Bedford, MA., Shimadzu Corporation, Columbia, MD, Hewlett-Packard Corporation, Avondale, PA, Rheodyne, Inc., Cotati, California, and others.

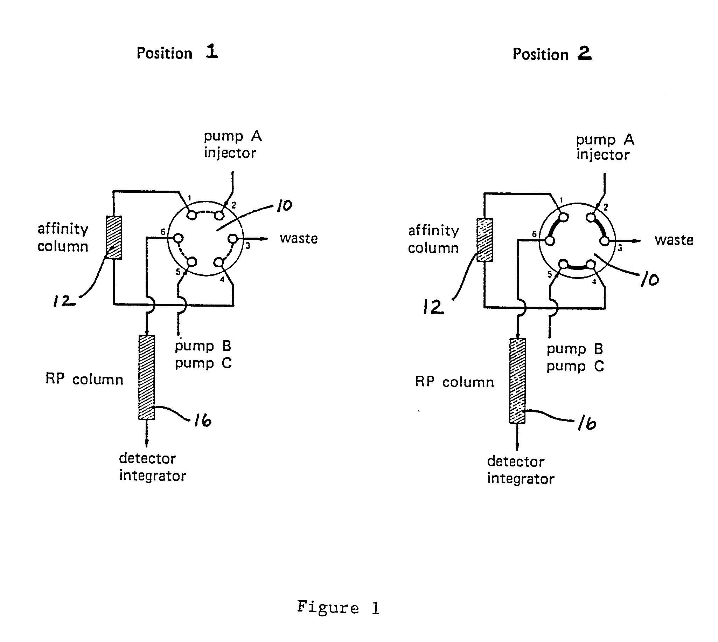

A schematic diagram of an exemplary valving configuration suitable for performing the assay of the present invention in an automated manner is shown in Figure 1. As shown therein, switching valve 10 has positions 1 and 2 illustrated by the left and right hand portions of the figure, respectively. In position 1, pump A and its associated injector (not shown) are connected to affinity column 12, which, in turn, is connected to a waste receptacle (not shown), while pumps B and C (not shown) are connected to reversed-phase (RP) column 16, whose output passes to an optical sensor and integrator (not shown). In this position, the affinity and reversed-phase columns are isolated from one another so that operations can be performed on each of the columns separately. In position 2, on the other hand, the affinity and reversed-phase columns are connected in series to one another and to pumps B and C. This position of valve 10 is used when material is to be transferred from the affinity column to the reversed-phase column.

A typical procedure for performing the assay of the present invention with the equipment of Figure 1 is as follows. With valve 10 in position 1, affinity column 12 is equilibrated by means of pump A with a suitable blocking buffer, e.g., a sodium sulfate/sodium phosphate buffer containing albumin, having a pH such that binding between interferon and the immobilized antibodies on the column will occur, e.g., a pH of about 6.8.

Once the column has been equilibrated, the sample to be analyzed, whose pH has been adjusted, if necessary, to a pH similar to that of the blocking buffer, is loaded onto the column by means of pump A and its associated injector.

With valve 10 still in position 1, the affinity column is washed to removed non-interferon components of the sample. A typical washing protocol can comprise, for example, a wash with the loading buffer, followed by a wash with a washing buffer containing, for example, ethylene glycol to facilitate removal of non-interferon proteins from the affinity column, followed by a further wash with the loading buffer.

While the washing is being performed the reversed-phase column is equilibrated with an aqueous solvent/organic solvent mixture selected to ensure strong uptake of interferon on the column (hereinafter the "RP equilibration buffer"). For example, for recombinant $\alpha2$ interferon, a $C_4$ column, and the preferred TFA/water/acetonitrile solvent system described above, it has been found suitable to equilibrate the RP column with an 85/15 or 80/20 mixture of 0.1% TFA in HPLC quality water (hereinafter the "RP flushing buffer") and 0.1% TFA in HPLC quality acetonitrile (hereinafter the "RP eluting buffer"). Other solutions of course can be used to equilibrate the RP column and will be readily apparent to persons skilled in the art in view of the present disclosure.

After washing of the affinity column and equilibration of the RP column have been completed, valve 10 is switched to position 2 and an eluting buffer is passed through the affinity column and into the RP column by means of either pump B or pump C. The eluting buffer breaks the bonds between the interferon and the affinity column's immobilized antibodies and carries the free interferon from the affinity column to the RP column. This buffer can contain chaotropic ions, capable of disassociating antigen-antibody complexes, such as, thiocyanate, perchlorate, or iodide ions. More preferably, the disassociation is achieved by simply using a buffer having a low pH. For example, a sodium sulfate/TFA buffer having a pH of about 2.4 has been found to successfully disassociate recombinant $\alpha2$ interferon from LIT-1 monoclonal antibodies.

After the elution from the affinity column and the transfer to the RP column have been completed, valve 10 is switched back to position 1. Using pumps B and C, the RP column is then re-equilibrated with the RP equilibration buffer (see above). Elution of the interferon from the RP column is then performed by passing an increasing gradient of the organic solvent through the column, e.g., a gradient increasing from 85% flushing buffer/15% eluting buffer to 100% eluting buffer. The amount of protein leaving the column as a function of time is monitored, recorded, and, as appropriate, integrated values corresponding to total protein or individual peaks are computed. As described above, the integrated values are converted to interferon quantities by means of a standard curve obtained by conducting the assay under the same conditions on samples containing known amounts of interferon.

At the same time elution is being performed on the RP column, the affinity column is prepared for the next assay. Using pump A, an albumin-containing blocking buffer is passed through the column to block any exposed silanol groups, following which the column is ready to perform the next assay.

In general, it is preferred to keep samples to be assayed at a low temperature, e.g., a temperature of about 5°C, so that they will not degrade. The remaining components of the system can be kept at room temperature.

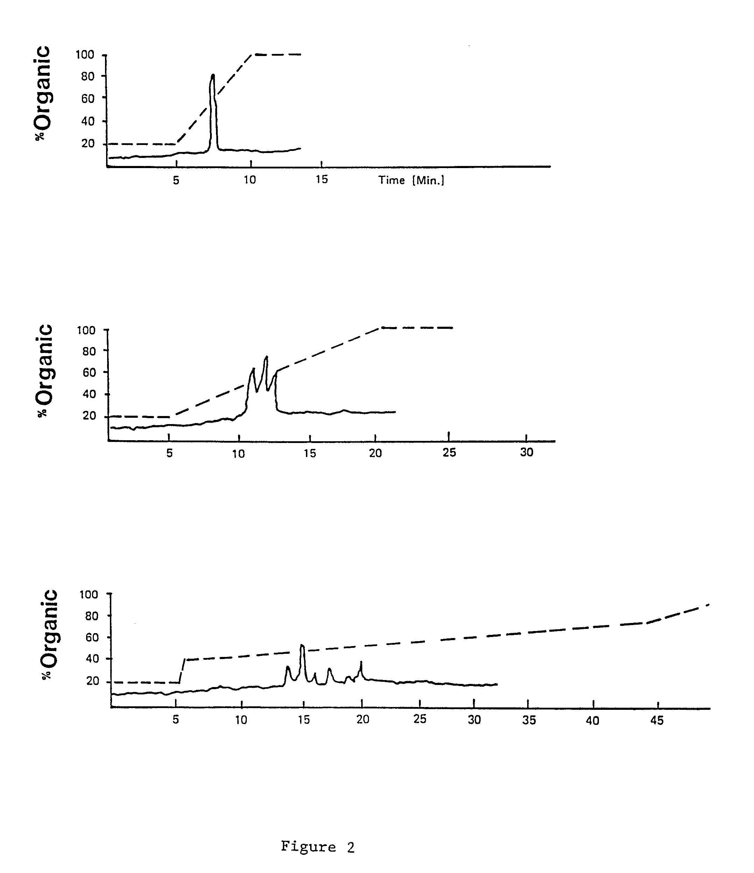

The resolution of the assay, i.e., its ability to separately detect and quantify different subtypes of interferon, is determined by the shape of the organic solvent gradient used to elute the interferon from the reversed-phase column. This dependence is illustrated schematically in Figure 2, where the dotted line represents the gradient of the organic solvent and the solid line the level of UV absorbance observed at the output of the RP column. (The times shown are typical of those employed with the equipment and materials described above.) As shown in this figure, if only total interferon quantities need to be determined, a fast gradient can be used, thus reducing the amount of time needed to perform the assay. On the other hand, if detailed information regarding interferon subtypes is needed, a slower gradient is required.

Based on the foregoing description, the invention will now be further illustrated by the following specific examples.

Example 1

Preparation of Affinity Column

This example illustrates the preparation of an affinity column suitable for use in assaying samples containing α2 interferon in accordance with the procedure of the present invention.

Monoclonal antibody was immobilized on a silica support following the procedures described by Roy et al., J. Chromatography, 303:225 (1984), and Ohlson et al., FEBS Letters, 93:5 (1978). Four grams of polyhydroxy silica particles (Nu-Gel PNP, 500 angstrom pore size, 200/400 mesh, Diagnostic Specialities, Metuchen, NJ) were suspended in distilled water and fines removed by repeated decantation of the turbid supernatant. (GP-200, 40 micron polyhydroxylated silica particles from Sota Chromatography, Crompond, NY can be used in place of the Nu-Gel particles.) Five milliliters of distilled water containing 100 mg of sodium periodate (Sigma, St. Louis, MO) were added to the wet silica cake. The mixture was agitated for 30 minutes at room temperature and then quickly collected on a coarse sintered glass filter.

The activated particles were immediately suspended in 11 milliliters of a monoclonal antibody solution containing LIT-1 antibody, which has been purified by anion-exchange chromatography. The antibody solution contained 40 milligrams of proteins and had been dialyzed against a coupling buffer (0.1 M sodium phosphate, 0.1 M sodium chloride, pH 7.0) prior to use. Also added to the particle/antibody suspension were 3 milligrams of sodium cyanoborohydride (Aldrich, Milwaukee, WI). The suspension was agitated gently overnight at 4°C. The resulting gel was collected on a sintered glass filter and washed three times with coupling buffer. The protein content of the filtrate indicated nearly quantitative binding of the antibody to the silica. Coupling density was thus about 10 milligrams antibody per gram of dry particles.

The silica particles and their attached antibodies were then suspended in 40 milliliters of 1 M ethanolamine hydrochloride (Fisher, Springfield, NJ), containing 40 milligrams of sodium borohydride to block unreacted sites on the particles. The pH of this solution was approximately 8.1. The suspension was agitated overnight at 4°C, washed three times with coupling buffer, and then stored in distilled water containing 0.1% sodium azide at 4°C. To produce the desired affinity column, 0.6 milliliters of the particles were packed into a 0.5 x 5 cm glass column (Pharmacia, Piscataway, NJ).

Example 2

Assays for α2 Interferon

This example illustrates the use of the present invention to assay samples for α2 interferon and α2 interferon subtypes.

The procedures and equipment employed in this example were those described above in connection with Figure 1. Specifically, the apparatus used included a Waters automatic gradient controller (Model 680), a Waters Automated Valve Station (Model WAVS) which includes a 6-port valve, a Shimadzu autoinjector (Model SIL-6), and a Hewlett-Packard integrator (Model 3392A). The Shimadzu autoinjector initiated the overall assay process, while the Waters gradient controller controlled the operation of the integrator and the flow of buffers and sample through the affinity of RP columns.

The affinity column was prepared in accordance with the procedures of Example 1 and the RP column was a Vydac C-4, 0.46 x 15 cm column, packed with 5 micron particles (The Separations Group, Hesperia, CA). In some cases, a microbore column packed with the same or similar particles was used to perform the reversed-phase separation, e.g. Chromega C-4, 10 x 0.7 cm, 5 micron particle size, 300A pore size.

The buffers employed were as follows: loading buffer (affinity column) --0.2 M $Na_2SO_4$, 2 mM $NaH_2PO_4$, 100 ppm $NaN_3$, pH 6.8; washing buffer (affinity column) --0.4 M $Na_2SO_4$, 40% ethylene glycol, 200 ppm $NaN_3$, pH 6.8; eluting buffer (affinity column) --0.1 M $Na_2SO_4$, 0.2% TFA, pH approximately 2.4; blocking buffer (affinity column) --0.2 M $Na_2SO_4$, 2 mM $NaH_2PO_4$, 200 ppm $NaN_3$, 0.7 mg/ml HSA, pH 6.8; flushing buffer (RP column) --0.1% TFA in water; and eluting buffer (RP column) --0.1% TFA in acetonitrile.

Prior to being assayed, clear samples were diluted with the loading buffer to a 5-20 microgram/milliliter interferon protein level. Cell lysates taken directly from the interferon production process were diluted with the loading buffer to the same interferon levels, and then centrifuged at 100,000 rpm (400,000 g) for 2 minutes (Beckman Ultracentrifuge TL-100, rotor model TLA-100.3). The resulting supernatant was then filtered through a 0.45 micron filter. Where necessary, the pH levels of the samples were adjusted to between 6 and 8.

Standards were prepared by dissolving accurately measured quantities of purified, lyophilized α2 interferon, which had been dried to a constant weight over magnesium perchlorate, in the loading buffer to produce a master standard having a protein concentration in the range of 1 milligram interferon per gram of solution. Working standards having concentrations in the range from 2-20 micrograms/milliliter were prepared from the master standard. All standards were aliquoted and stored at -70°C.

With the 6-port valve in its first position (see Figure 1) samples and standards (sample size 1 ml) were loaded onto the affinity column (previously equilibrated with the blocking buffer) via the autoinjector. The affinity column was washed sequentially with 18 column volumes of the loading buffer, 44 column volumes of the wash buffer, and 20 column volumes of the loading buffer. Simultaneously, the RP column was equilibrated with an 85/15 mixture of the RP flushing and RP eluting buffers.

After switching of the 6-port valve to its second position, the interferon on the affinity column was eluted onto the RP column with 8 column volumes of the affinity column eluting buffer. The 6-port valve was then returned to its first position and the affinity column was flushed with 6 column volumes of the blocking buffer in order to bring it to neutrality and to block residual silanol groups.

Simultaneously, interferon was eluted from the RP column. First, the RP column was flushed with 15 ml of an 85/15 mixture of the RP flushing buffer and the RP eluting buffer. Then, over a period of 10 minutes, the buffer flowing through the RP column was linearly changed from the 85/15 mixture to 100% eluting buffer.

The flow rates used during loading, washing, elution, and blocking of the affinity column were 0.5 ml/min, 2 ml/min, 1.4 ml/min, and 0.7 ml/min, respectively. The flow rates used during elution of the RP column were 1.4 ml/min for the 85/15 mixture, 0.7 ml/min while the percentage of eluting buffer was increasing, and 2 ml/min once the 100% level of eluting buffer had been reached.

Using these flow rates, the total time required for an assay, including equilibration of both columns, was on the order of 45 minutes. These flow rates have been found to cause no apparent deterioration in the performance of either of the columns, even after several hundred assays.

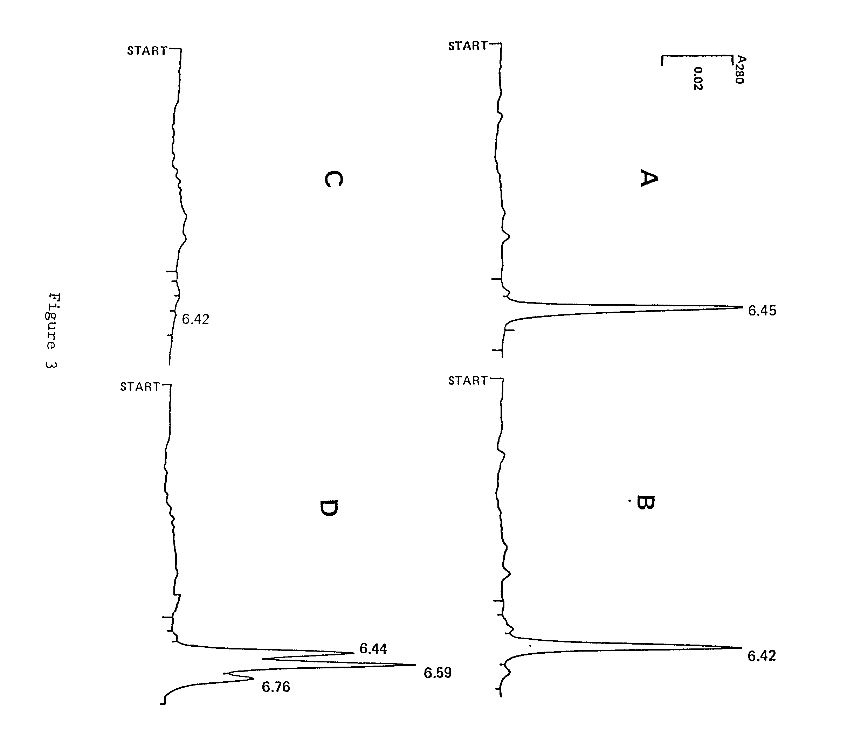

Typical assay results achieved with the present assay are shown in Figure 3, where the sample for panel A was the loading buffer spiked with α2 standard; for panel B, yeast lysate from yeast cells which did not include an α2 interferon gene, spiked with the same standard; for panel C, the same lysate without spiking; and for panel D, yeast lysate from yeast cells containing an α2 interferon gene. Significantly, as shown by a comparison of panels A-C, yeast lysate proteins do not elute from the RP column along with the α2 interferon and thus do not interfere with the interferon assay.

As demonstrated by panel D, the assay readily distinguishes between different interferon subtypes. Subsequent analysis of the three peaks in this panel has shown that the first peak contains mostly fully oxidized interferon monomers having intact disulfide bonds, the second peak is enriched in partially oxidized monomers having some disulfide bonds broken, and the third peak is associated with interferon oligomers.

In addition to the results shown in Figure 3, a SDS/PAGE analysis has been performed on the load material, elution peaks, and unbound peaks for yeast lysates with and without interferon genes and with and without interferon spiking (data not shown). These analyses show typical patterns of homogeneous interferon bands and the absence of non-interferon proteins in the elution peaks. Comparisons of the load and elution peaks for interferon standards have shown identical band patterns, thus demonstrating that the assay does not change the interferon composition of samples being assayed.

A typical standard curve for the assay is shown in Figure 4. As can be seen from this curve, the response of the assay is linear over its dynamic range of from about 1 microgram/milliliter to about 20 micrograms/milliliter. Repeated analyses of the same standard have shown that the relative standard deviation of the assay is on the order of 5%.

The overall sensitivity of the assay can be further improved by using the affinity column/RP column technique to enrich trace levels of interferon in samples and by then using other methods, e.g., immunoassays, fluorescent assays, or other techniques now known or subsequently developed, to quantify the amount of interferon in the eluate from the reversed-phase column. In this way, the assay of the present invention can be used in the quantification of the amounts of interferon in, for example, blood samples taken from patients undergoing interferon therapy.

As the foregoing data makes clear, the assay of the present invention can readily and accurately assay complex mixtures of proteins to determine the composition and concentration of interferons contained therein, e.g., crude yeast lysates, cell extracts, cell culture media, formulated products with excipients, E. coli extracts, etc.

Although specific embodiments of the invention have been described and illustrated, it is to be understood that modifications can be made without departing from the invention's spirit and scope. For example, apparatus other than that discussed and illustrated in Figure 1 can be used to practice the invention. Similarly, other loading, washing, eluting, blocking, and equilibrating protocols, as well as other buffer systems and affinity and RP columns, can be employed in the practice of the invention.

## Claims

1. A method for detecting interferon in a solution which contains both an interferon and a non-interferon component comprising the steps of:

(a) passing the solution through a column, containing an anti-interferon antibody coupled to a solid support so as to bond a portion of the interferon component in the solution to the anti-interferon antibody;

(b) passing a first buffer through the column to remove substantially all of the non-interferon component of the solution from the column without removing a substantial amount of the interferon component which has become bound to the antibody;

(c) passing a second buffer through the column to disrupt the interferon-antibody bonds and thus form an intermediate solution comprising a portion of the interferon component dissolved in the second buffer;

(d) passing the intermediate solution through a second column containing a solid support to which is bonded a hydrophobic material so that a portion of the interferon component in the intermediate solution is separated from the solution and becomes bound to the second column;

(e) eluting the bound interferon component from the second column by passing an increasing gradient of one or more organic solvents through the second column; and

(f) sensing the amount of protein in the eluate from the second column as a function of time.

2. The method of Claim 1 wherein the solid support for the anti-interferon antibody is made of silica and wherein the method includes the additional step of passing a protein-containing buffer through the antibody column prior to performing step (a).

3. The method of Claim 2 wherein the protein is albumin.

4. The method of Claim 3 wherein the albumin is bovine serum albumin.

5. The method of Claim 3 wherein the albumin is human serum albumin.

6. The method of Claim 1 wherein the anti-interferon antibody is a monoclonal antibody.

7. The method of Claim 1 wherein the hydrophobic material comprises $C_4$ groups.

8. The method of Claim 1 wherein the interferon is $\alpha 2$ interferon.

9. The method of Claim 1 including the additional step of quantifying the amount of interferon in the solution by comparing the amount of protein sensed in step (f) with a standard curve obtained by performing steps (a) through (f) on solutions containing known amounts of interferon.

10. The method of Claim 9 wherein amounts of interferon less than 30 micrograms per milliliter are quantified.

11. The method of Claim 10 wherein amounts of interferon on the order of 2 micrograms per milliliter are quantified.

12. The method of Claim 1 wherein the interferon component of the solution includes at least two subcomponents and the increasing gradient of one or more organic solvents causes the subcomponents to elute from the second column at different times.

13. The method of Claim 12 wherein the increasing gradient is a linear gradient.

14. The method of Claim 12 wherein the interferon is α2 interferon and the at least two subcomponents comprises a first eluting subcomponent, a second eluting subcomponent, and a third eluting subcomponent, the first eluting subcomponent being characterized by an enhanced concentration, in comparison with the second and third subcomponents, of fully oxidized interferon monomers, the second eluting subcomponent being characterized by an enhanced concentration, in comparison with the first and third subcomponents, of partially or fully reduced interferon monomers, and the third eluting subcomponent being characterized by an enhanced concentration, in comparison with the first and second subcomponents, of interferon oligomers.

Position **1**                    Position **2**

Figure 1

Figure 2

Figure 3

Slope =          .4169853

Intercept =      −6.493465E-03

Corr. Coeff. = .9985181

Area

microgroms/milliliter

Figure 4